# EUROPEAN PATENT SPECIFICATION

(11) **EP 1 927 335 B1**
(45) Date of publication and mention of the grant of the patent: **21.10.2009**
(21) Application number: 07022067.8
(22) Date of filing: 14.11.2007
(51) Int. Cl.: A61K 8/03, A61K 8/11, A61K 8/26, A61K 8/29, A61Q 5/12

(54) **Two-phase conditioning composition**
Zweiphasige Haarspülungszusammensetzung
Composition à deux phases pour le conditionnement

(30) Priority: 24.11.2006 EP 06024378
(43) Date of publication of application: 04.06.2008
(73) Proprietor: KPSS-Kao Professional Salon Services GmbH, 64297 Darmstadt (DE)
(72) Inventor: Hoffmann, Martin, 64673 Zwingenberg (DE); Tietjen, llka, 69207 Sandhausen (DE); Bräutigam, Ina, 64297 Darmstadt (DE)

(56) References cited:
- WO-A-02/060397
- WO-A-20/05065632
- DE-U1- 9 216 886

## Description

The present invention relates to a conditioning composition for keratin fibres especially hair comprising two physically separated phases at zero shear rate.

Conditioning compositions have been known for many years. There have been products on the market in various forms and for various benefits. Shine enhancing of hair together with other hair care benefits are increasingly been found as main product benefit. Among other products, products with two physically separated phases at zero share rate have recently been put onto market.

For example WO 02/060397 discloses dual phase conditioning and styling composition for heat styling hair. According to the document compositions comprise an oil phase comprising volatile oil and an aqueous alcoholic phase comprising styling polymer, an emulsifier and salt. The document does not address at all shine enhancing of hair and especially does not disclose any composition which may fall within the scope of the present invention.

EP 996 408 discloses dual phased compositions for make up cleansing wherein a polyvinylpyrrolidone copolymer is used as a demixing agent. The documents does not address at all hair care application of such composition.

The inventors of the present invention have surprisingly found out that a two phase composition comprising an oil phase and a water phase wherein two phases are optically separated at zero shear rate and becomes homogeneous upon shaking and returns again to optically separated two phases upon release of agitation conditions hair and especially enhances hair shine, smoothness and elasticity excellently.

Accordingly, the object of the present invention is a two phase composition for hair comprising 5 to 50%, by weight oil phase, 50 to 95%, by weight water phase and 0.001 to 10% by weight, all values are calculated to total composition, synthetic mica coated with metal oxide or oxides and having a volume particle size distribution in the range of 1 to 750 µm, wherein oil and aqueous phases are optically separated at zero shear rate and becomes homogeneous upon shaking and returns again to optically separated two phases upon release of agitation.

Compositions of the present invention comprises optically separated two phases which becomes homogeneous upon shaking and returns again to optically separated two phases upon release of agitation. Compositions of the present invention returns to optically separated two phases upon release of agitation preferably within 24 hours, more preferably within 20 hours and most preferably within 10 hours. It should be noted that with the compositions of the present invention separated oil and aqueous phases are recognizable after 15 to 30 minutes of shaking. The above mentioned separation periods refer to the complete separation of the two phases i.e. returning to their original state.

Compositions of the present invention comprises oil phase at a concentration of 5 to 50%, preferably 10 to 40% more preferably 15 to 30% by weight calculated to total composition. Oil phase comprises 70 to 100%, preferably 80 to 100% and more preferably 90 to 100% by weight calculated to the content of oil phase at least one volatile oil. Suitable volatile oils are silicones such as dimethicone and cyclomethicone and mixtures thereof, and volatile hydrocarbons such as isododecane, isohexadecane and isoprafin. The most preferred are silicones and from those dimethicone and cylomethicone and mixtures thereof.

Suitable volatile dimethicones are the ones with a viscosity of less than 5 mm²/s. The most preferred are dimethicone with a viscosity of 1 mm²/s and 0.65 mm²/s which are available from Dow Corning with the trade name DC 200 Fluid.

Suitable volatile cyclomethicones are according to general formula 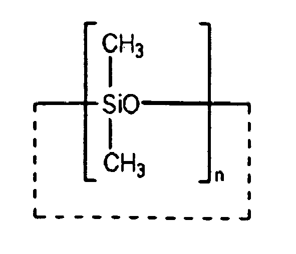 where n is a number between 3 and 7. Preferred are cyclopentasiloxanes known with the trade name for example Dow Corning 245.

Non-volatile silicones may also be incorporated into the compositions of the present invention at a low concentration, i.e. lower than 2%, preferably in the range of 0.01 to 1% by weight calculated to the content of oil phase. Suitable ones are arylated silicones such as phenyltrimethicone known with the trade name Dow Corning 556, and dimethicones and dimethiconol with higher viscosity available from Dow Corning under the trade name DC 200 fluid with higher viscosity values.

In addition, natural oils such as olive oil, almond oil, avocado oil, ricinus oil, jojba oil, coconut oil, palm oil, sesame oil, peanut oil, whale oil, sunflower oil, peach kernel oil, wheat germ oil, macadamia nut oil, night primrose oil, castor oil, or soya oil or their mixture, and mineral oil such as paraffin oil or petrolatum may suitably be contained. Concentration of natural oils are below 2%, preferably in the range of 0.01 to 1.0% by weight calculated to the content of the oil phase. As a rule selected natural oil should be soluble in and/or miscible with the volatile oil used in the composition.

Further compositions of the present invention can comprise fatty acid esters as non-volatile oily compounds such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc. at the same concentration range as the natural oils.

Compositions of the present invention comprise aqueous phase at a concentration of 50 to 95%, preferably 60 to 90% more preferably 70 to 85% by weight calculated to total composition.

Compositions of the present invention comprise synthetic mica coated with metal oxide or oxides and having a volume particle size distribution in the range of 1 to 750 µm. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and known with their INCl names Synthetic Fluorphologopite.

The particle size distribution of synthetic mica coated with a metal oxide or oxides is in the range of 1 to 750 µm, preferably 1 to 250 µm, more preferably 1 to 100 µm and most preferably 20 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.001 to 10%, preferably 0.01 to 7.5%, more preferably 0.05 to 5% and most preferably 0.1 to 2.5% by weight calculated to total composition.

With the term volume particle size distribution it is meant that synthetic mica coated with a metal oxide or oxides include particle with a size in the range of 1 to 750 µm measured as volume diameter.

Synthetic mica is preferably added as dispersed in water phase. It has been observed that the synthetic mica particles coated with metal oxide or oxides migrate to the water and oil interphase. This does not effect the performance of the composition as shaking is required prior to application. This phenomenon should also not be seen as the formation of the third phase as particles migrated to the oil-water interphase are still dispersed in water and contained in the water phase.

In a preferred embodiment of the present invention, compositions comprise at least one hair conditioning compound in the aqueous. Suitable hair conditioning compounds are those water soluble and/or water miscible compounds such as cationic surfactants, cationic polymers, polyols and natural plant extracts. It should be noted that cationic polymers can also be used as hair fixing agent.

One or more cationic surfactant(s) as hair conditioners is (are) contained in aqueous phase of the compositions of the present invention at a concentration of 0.01 to 2%, preferably 0.05 to 1.5%, more preferably 0.1 to 1.0% by weight calculated to the content of the aqueous phase. Suitable cationic surfactants are according to the general formula 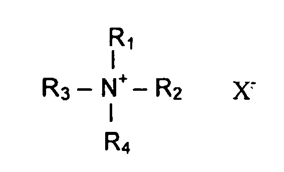 where R₁ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅ CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms, ethoxy or propoxy group with number of ethoxy or propoxy groups varying in the range of 0 to 4and X is chloride, bromide or methosulfate.

Suitable cationic surfactants are particularly cetyl trimethly ammonium chloride, steartrimonium chloride, behentrimoinium chloride, stearamidopropyl trimonuim chloride, behenamidopropylethyldimonium ethosulfate, behenamidopropyltrimonium methosulfate, cocamidopropyltrimonium chloride, cocotrimonim chloride, palmitamidopropyltrimonum chloride, dipalmitoyltrimonium chloride, di-C12-C15 alkyldimoniumchloride, distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride and dipalmitoylethyldimonium chloride.

One or more polyol(s) as hair conditioners is (are) comprised in the aqueous phase of the compositions of the present invention at a concentration of 0.01 to 10%, preferably 0.05 to 7.5%, 0.1 to 5% by weight calculated to total of aqueous phase. Suitable ones are panthenol, glycerol, polyethylene glycols with molecular weight 200 to 20,000.

Natural plant extracts may as well form part of the compositions of the present invention. Natural extracts should be included into the aqueous phase of the compositions. Suitable aqueous (e.g. steam-distilled) alcoholic or hydro-alcoholic plant extracts known per se are in particular extracts from leaves, fruits, blossoms, roots, rinds or stems of almond, aloe, pineapple, artichoke, arnica, avocado, valerian, henbane, birch, stinging nettle, echinacea, ivy, wild angelica, gentian, ferns, pine needles, silver weed, ginseng, broom, bamboo, green tea, oat, rose hip, hamamelis, hay flowers, elderberry, hop, coltsfoot, currants, chamomile, carrots, chestnuts, clover, burr root, coconut, cocoanut, mango, peach, lemon, cornflower, wheat, apricot, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

In another preferred embodiment of the present invention compositions comprise one or more hair fixing polymers in aqueous phase at a concentration of 0.1 to 20%, preferably 0.5 to 15%, more preferably 0.5 to 10 % by weight calculated to the content of aqueous phase.

Suitable polymers are non-ionic, cationic, amphoteric and anionic polymers. One of the important point in selecting fixing polymer is that the polymer should not thicken the aqueous phase. In other words the viscosity of the aqueous phase should remain low i.e. below 100 mPa.s, preferably 50 mPa.s measured at 20°C.

Suitable non-ionic polymers are polyvinylpyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer. In addition to these fixing polymers derivatives of natural polymers such as hydroxyl ethylcellulose may also be used in combination with one of the fixing polymers mentioned above.

Suitable cationic polymers include Polyquaternium-4, Polyquaternium-11, Polyquaternium-16, Polyquaternium-18, Polyquaternium-24, Polyquaternium-28, Polyquaternium-46, polyvinylpyrrolidone/dimethylaminoethyl methacrylate, and cationically derivatized natural polymers. Preferred are Polyquaternium-4, Polyquaternium-11 and Polyquaternium-16.

Suitable amphoteric polymers are those available from the company National Starch under the trade name Amphomer such as Octylacrylamide/Acrylates/Butylaminoethyl methacrylate copolymer, those available under the trade name Diaformer (methacryloylethylbetaine/methacrylates copolymer). Preferred are the ones available under the trade name Amphomer.

As a fixing polymer silicone containing polymers either grafted or block copolymer are also useful. Suitable and the preferred one is Polysilicone-9.

Aqueous phase of the compositions of the present invention comprises 5 to 40%, preferably 10 to 35%, more preferably 15 to 30% by weight calculate to the content of aqueous phase at least one water miscible organic solvent-. With the term "water miscible" it is meant that the organic solvent is miscible with water at any ratio. Suitable ones are C₂ - C₄ monohydric alcohols such as ethanol, propanol, isopropanol, butanol and isobutanol, benzyl alcohol and benzyloxyethanol and their mixtures. Most preferred are ethanol and isopropanol.

Compositions of the present invention can comprise UV filters for protection of hair from environmental influences. It should be noted that oil soluble UV filters are contained in the oil phase and water soluble ones are added to the aqueous phase although partitioning the UV filter between two phases is not excluded. The UV-absorbing substance is preferably selected from the following compounds: Polysiliocne-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxybenzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzylidenecampher, 3-(4'-sulfo)-benzyl-idenebornane-2-one and the salts thereof, 3-(4'-methyl benzylidene)-DL-campher and/or 2-ethylhexyl-2-cyano-3,3-diphenyl acrylate also known as Octocrylene. The preferred amount of the UV-absorber ranges from about 0.01 % to 2.5%, more preferably from 0.05 % to 1 % by weight, calculated to the total composition. Needless to mention that water soluble UV filters should be added to the water phase band the oil soluble ones into oil phase.

The pH of the aqueous phase varies from 3 to 7, particularly 4.5 to 6. For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, pyruvic acid, salicylic acid or acetic acid or inorganic acids such as hydrochloric acid, phosphoric acid, sulphuric acid, nitric acid. The pH of the conditioner composition can also be adjusted to the required pH by using alkaline solution such as sodium hydroxide, potassium hydroxide, ammonium hydroxide or their salts with those acids mentioned above.

The aqueous and oil phases of the compositions of the present invention may be coloured. The dyestuff suitable for product colouring purposes are all useful for this purpose. It should be noted that for colouring aqueous phase water soluble dyes should be used and for colouring oil phase oil soluble dyes are suitable. The nature of the dyestuff is actually not important but preferred are non substantive dyes, i.e. not remaining on hair after washing with water or shampooing.

Fragrance, chelating agent, preservatives and other conventional cosmetic ingredients can be included at their usual concentrations either into the oil or aqueous phases depending on their solubility.

Composition of the present invention are leave-in composition, i.e. not rinsed off from hair after application. Accordingly, in a process compositions of the present invention are applied to the shampooed and/or wetted hair and hair is dried. Compositions of the present invention are also applied onto dry hair. It is possible to apply compositions of the present invention as a lotion and also from a bottle equipped with a pump spray. Preferred application is spraying onto hair.

The compositions of the present invention may be transparent or turbid. Turbidity may be caused by the presence of synthetic mica particles or any other ingredient.

Following examples are to illustrate the invention, but not to limit.

### Example 1

| Oil phase | |
|---|---|
| | % by weight |
| Trisiloxane* | 99.8 |
| Fragrance | 0.2 |

| | |
|---|---|
| *: DC 200 Fluid with a viscosity of 1 cst | |

| Aqueous phase | |
|---|---|
| | % by weight |
| Synthetic fluorphologopite** | 0.5 |
| Citric acid/Ammonium hydroxide | q.s. to pH 6.0 |
| Water | to 100 |

| | |
|---|---|
| **: Synthetic fluorphologopite used is commercially available from Sun Chemical Corporation under the trade name SunShine Glitter White with a particle size distribution in the range of 20 to 95 µm. | |

The above composition is used at a concentration of oil phase 20% by weight and aqueous phase 80% by weight, calculated to total composition.

For the comparative purposes the same composition without Synthetic fluorphologopite was also prepared.

The two compositions were compared in a half side test with 10 female volunteers and it was observed that the side treated with inventive composition was significantly more shiny, has better elasticity and felt smoother upon touching. Preference was 9/1, wherein in 1 case there was no difference seen. The composition was applied form a bottle with a spraying device after shaking to homogeneity.

### Example 2

| Oil phase | |
|---|---|
| | % by weight |
| Trisiloxane* | 69.4 |
| Cyclopentasiloxane | 30.0 |
| Isopropyl myristate | 0.3 |
| Ethylhexylmethoxycinnamate | 0.1 |
| Fragrance | 0.2 |

| | |
|---|---|
| *: DC 200 Fluid with a viscosity of 1 cst | |

| Aqueous phase | |
|---|---|
| | % by weight |
| Polyqauternium-11 | 0.5 |
| Synthetic fluorphologopite** | 0.5 |
| Steartrimoniumchloride | 0.15 |
| Ethanol | 20.0 |
| Lactic acid/Ammonium hydroxide | q.s. to pH 6.0 |
| Water | to 100 |

| | |
|---|---|
| **: Synthetic fluorphologopite used is commercially available from Sun Chemical Corporation under the trade name SunShine Glitter White with a particle size distribution in the range of 20 to 95 µm. | |

Similar effects were observed when used on hair as in Example 1.

### Example 3

| Oil phase | |
|---|---|
| | % by weight |
| Cyclopentasiloxane | 99.5 |
| Dimethicone 350 cp | 0.3 |
| Fragrance | 0.2 |

| Aqueous phase | |
|---|---|
| | % by weight |
| VP/VA copolymer | 0.5 |
| Synthetic fluorphologopite* | 0.3 |
| Ethanol | 20.0 |
| Lactic acid/Ammonium hydroxide | q.s. to pH 6.0 |
| Water | to 100 |

| | |
|---|---|
| *:Synthetic fluorphologopite used is commercially available from Sun Chemical Corporation under the trade name SunShine Glitter White with a particle size distribution in the range of 20 to 95 µm. | |

Similar effects were observed when used on hair as in Example 1.

### Example 4

| Oil phase | |
|---|---|
| | % by weight |
| Trisiloxane* | 99.5 |
| Dimethicone 350 cp | 0.3 |
| Fragrance | 0.2 |

| | |
|---|---|
| *:DC 200 Fluid with a viscosity of 1 cst | |

| Aqueous phase | |
|---|---|
| | % by weight |
| VP/VA copolymer | 0.5 |
| Synthetic fluorphologopite** | 0.3 |
| Ethanol | 20.0 |
| Benzophenone-4 | 0.2 |
| Citric acid/Sodium hydroxide | q.s. to pH 6.0 |
| Water | to 100 |

| | |
|---|---|
| **: Synthetic fluorphologopite used is commercially available from Sun Chemical Corporation under the trade name SunShine Glitter White with a particle size distribution in the range of 20 to 95 µm. | |

Similar effects were observed when used on hair as in Example 1.

### Example 5

| Oil phase | |
|---|---|
| | % by weight |
| Trisiloxane* | 99.7 |
| Almond oil | 0.1 |
| Fragrance | 0.2 |

| | |
|---|---|
| *:DC 200 Fluid with a viscosity of 0.65 cst | |

| Aqueous phase | |
|---|---|
| | % by weight |
| Polyquaternium 16 | 0.5 |
| Cetrimonium chloride | 0.5 |
| Synthetic fluorphologopite** | 0.3 |
| Ethanol | 15.0 |
| Benzophenone-4 | 0.2 |
| Citric acid/Sodium hydroxide | q.s. to pH 6.0 |
| Water | to 100 |

| | |
|---|---|
| **: Synthetic fluorphologopite used is commercially available from Sun Chemical Corporation under the trade name SunShine Glitter White with a particle size distribution in the range of 20 to 95 µm. | |

Similar effects were observed when used on hair as in Example 1.

### Example 6

| Oil phase | |
|---|---|
| | % by weight |
| Dimethicone 1 cst | 99.6 |
| Benzophenone-3 | 0.2 |
| Fragrance | 0.2 |

| Aqueous phase | |
|---|---|
| | % by weight |
| VP/VA copolymer | 3.0 |
| Polysilicone-9 | 0.5 |
| Cetrimonium chloride | 0.15 |
| Dioleylethyl hydroxyethylmonium chloride | 0.15 |
| Synthetic fluorphologopite | 0.6 |
| Benzophenone-4 | 0.10 |
| Ethanol | 25.0 |
| Lactic acid/Ammonium hydroxide | q.s. to pH 7.0 |
| Dyestuff CI 19140 and CI 42053 | q.s. |
| Water | to 100 |

| | |
|---|---|
| **: Synthetic fluorphologopite used is commercially available from Sun Chemical Corporation under the trade name SunShine Glitter White with a particle size distribution in the range of 20 to 95 µm. | |

Similar effects were observed when used on hair as in Example 1.

## Claims

1. Two phase composition for hair **characterised in that** it comprises 5 to 50%, by weight calculated to total composition, oil phase, 50 to 95% by weight aqueous phase, calculated to total composition and 0.001 to 10% by weight, calculated to total composition, synthetic mica coated with metal oxide or oxides and having a volume particle size distribution in the range of 1 to 750 µm, wherein oil and aqueous phases are optically separated at zero shear rate and becomes homogeneous upon shaking and returns again to optically separated two phases upon release of agitation.

2. Composition according to claim 1 **characterised in that** it comprises in the oil phase 70 to 100% by weight calculated to the content of oil phase at least one volatile oil.

3. Composition according to claims 1 and 2 **characterised in that** it comprises in the oil phase non-volatile oil less than 2% by weight calculated to content of oil phase.

4. Composition according to claims 1 to 3 **characterised in that** it comprises at least one hair conditioning compound in the aqueous phase.

5. Composition according to any of the preceding claims **characterised in that** it comprises at least one hair fixing polymer in the aqueous phase.

6. Composition according to any of the preceding claims **characterised in that** it comprises at least one water miscible organic solvent at a concentration of 5 to 40% by weight, calculated to content of aqueous phase.

7. Composition according to any of the preceding claims **characterised in that** it comprises at least one UV filter.

8. Composition according to any of the preceding claims **characterised in that** aqueous phase has a pH between 3 and 7.

9. Composition according to any of the preceding claims **characterised in that** it is a leave-in composition

10. Use of the composition according to any of the preceding claims for conditioning hair.

## Patentansprüche

1. Zweiphasen-Zusammensetzung für Haare, **dadurch gekennzeichnet, dass** 5 bis 50 Gew.- % Ölphase, 50 bis 95 Gew.- % wässrige Phase und 0,001 bis 10 Gew.- %, jeweils berechnet auf die Gesamtzusammensetzung, synthetisches Mica, das mit Metalloxyd oder Oxyden beschichtet ist und eine Partikelgrößenverteilung im Bereich von 1 bis 750 µm hat, enthalten ist, worin die Öl- und die wässrige Phase in der null Shear-Rate optisch getrennt sind, nach dem Schütteln homogen gemischt werden und nach der Beendigung des Schüttelns wieder in die optisch separaten zwei Phasen zurückkehren.

2. Zusammensetzung nach Anspruch 1, **dadurch gekennzeichnet, dass** in der Ölphase 70 bis 100 Gew.- % mindestens eines flüchtigen Öles, berechnet auf den Gesamtgehalt der Ölphase, enthalten ist.

3. Zusammensetzung nach den Ansprüchen 1 und 2, **dadurch gekennzeichnet, dass** in der Ölphase weniger als 2 Gew.- % nichtflüchtiges Öl, berechnet auf den Gesamtgehalt der Ölphase, enthalten ist.

4. Zusammensetzung nach den Ansprüchen 1 bis 3, **dadurch gekennzeichnet, dass** mindestens ein Haarkonditioniermittel in der wässrigen Phase enthalten ist.

5. Zusammensetzung nach einem der vorhergehenden Ansprüche, **dadurch gekennzeichnet, dass** mindestens ein Haar festigendes Polymer in der wässrigen Phase enthalten ist.

6. Zusammensetzung nach einem der vorhergehenden Ansprüche, **dadurch gekennzeichnet, dass** mindestens ein mit Wasser mischbares organisches Lösungsmittel in einer Menge von 5 bis 40 Gew.- %, berechnet auf den Gesamtgehalt der wässrigen Phase, enthalten ist.

7. Zusammensetzung nach einem der vorhergehenden Ansprüche, **dadurch gekennzeichnet, dass** mindestens einem UV- Filter enthalten ist.

8. Zusammensetzung nach einem der vorhergehenden Ansprüche, **dadurch gekennzeichnet, dass** die wässrige Phase einen pH- Wert zwischen 3 und 7 hat.

9. Zusammensetzung nach einem der vorhergehenden Ansprüche, **dadurch gekennzeichnet, dass** sie eine Leave- in- Zusammensetzung ist.

10. Verwendung der Zusammensetzung nach einem der vorhergehenden Ansprüche zum Konditionieren von Haaren.

## Revendications

1. Composition biphasique pour les cheveux **caractérisée en ce qu'**elle comprend 5 à 50%, en poids calculé par rapport à la composition totale, de phase huileuse, 50 à 95% en poids de phase aqueuse, calculé par rapport à la composition totale et 0,001 à 10% en poids, calculé par rapport à la composition totale, de mica synthétique enrobé d'oxyde ou d'oxydes métallique(s) et ayant une distribution de taille des particules en volume dans la gamme de 1 à 750 µm, dans laquelle les phases huileuse et aqueuse sont séparées optiquement à vitesse de cisaillement nulle et deviennent homogènes dès agitation et reviennent de nouveau à l'état de deux phases optiquement séparées à l'arrêt de l'agitation.

2. Composition selon la revendication 1, **caractérisée en ce qu'**elle comprend dans la phase huileuse 70 à 100% en poids calculé par rapport au contenu de la phase huileuse d'au moins une huile volatile.

3. Composition selon les revendications 1 et 2 **caractérisée en ce qu'**elle comprend dans la phase huileuse une huile non-volatile inférieure à 2% en poids calculé par rapport au contenu de la phase huileuse.

4. Composition selon les revendications 1 à 3 **caractérisée en ce qu'**elle comprend au moins un agent conditionneur pour les cheveux dans la phase aqueuse.

5. Composition selon l'une quelconque des revendications précédentes **caractérisée en ce qu'**elle comprend au moins un polymère de fixation des cheveux dans la phase aqueuse.

6. Composition selon l'une quelconque des revendications précédentes **caractérisée en ce qu'**elle comprend au moins un solvant organique miscible à l'eau à une concentration de 5 à 40% en poids, calculée par rapport au contenu de la phase aqueuse.

7. Composition selon l'une quelconque des revendications précédentes **caractérisée en ce qu'**elle comprend au moins un filtre UV.

8. Composition selon l'une quelconque des revendications précédentes **caractérisée en ce que** la phase aqueuse a un pH compris entre 3 et 7.

9. Composition selon l'une quelconque des revendications précédentes **caractérisée en ce qu'**elle est une composition sans rinçage.

10. Utilisation de la composition selon l'une quelconque des revendications précédentes pour le conditionnement des cheveux.
